(19) Europäisches Patentamt / European Patent Office / Office européen des brevets

(11) **EP 3 345 601 A1**

(12) **EUROPEAN PATENT APPLICATION**
published in accordance with Art. 153(4) EPC

(43) Date of publication:
**11.07.2018 Bulletin 2018/28**

(21) Application number: **16841954.7**

(22) Date of filing: **01.09.2016**

(51) Int Cl.:
$A61K\ 31/436^{(2006.01)}$    $A61K\ 47/22^{(2006.01)}$
$A61K\ 47/26^{(2006.01)}$    $A61K\ 47/38^{(2006.01)}$
$A61P\ 1/04^{(2006.01)}$    $A61P\ 1/16^{(2006.01)}$
$A61P\ 3/10^{(2006.01)}$    $A61P\ 11/00^{(2006.01)}$
$A61P\ 13/12^{(2006.01)}$    $A61P\ 17/00^{(2006.01)}$
$A61P\ 17/06^{(2006.01)}$    $A61P\ 19/02^{(2006.01)}$
$A61P\ 21/04^{(2006.01)}$    $A61P\ 25/00^{(2006.01)}$
$A61P\ 27/02^{(2006.01)}$    $A61P\ 29/00^{(2006.01)}$
$A61P\ 37/06^{(2006.01)}$

(86) International application number:
**PCT/JP2016/075617**

(87) International publication number:
**WO 2017/038925 (09.03.2017 Gazette 2017/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **03.09.2015 JP 2015173754**

(71) Applicant: **Nippon Kayaku Kabushiki Kaisha Tokyo 100-0005 (JP)**

(72) Inventor: **KAWAMURA Dai Tokyo 115-8588 (JP)**

(74) Representative: **Godemeyer Blum Lenze Patentanwälte Partnerschaft mbB - werkpatent An den Gärten 7 51491 Overath (DE)**

(54) **PHARMACEUTICAL COMPOSITION CONTAINING RAPAMYCIN OR DERIVATIVE THEREOF**

(57) It is an object of the present invention to provide a pharmaceutical composition, which can suppress a reduction in the content of an active ingredient caused by oxidation or decomposition of rapamycin or a derivative thereof, can ensure long-term stability, and has high safety. In the present invention, the inventors have found that (B) a salt of an ascorbic acid or a derivative thereof and/or a salt of chelating agent is used in a pharmaceutical formulation comprising rapamycin or a derivative thereof, so that oxidation or decomposition of the rapamycin or a derivative thereof can be suppressed, thereby completing the present invention. The rapamycin or a derivative thereof, and (B) the salt of an ascorbic acid or a derivative thereof and/or the salt of chelating agent are preferably in the form of a solid mixture produced by preparing a solution containing these components and then removing the solvent from the solution.

**EP 3 345 601 A1**

**Description**

Technical Field

The present invention relates to a pharmaceutical formulation composition comprising rapamycin or a derivative thereof, the stability of which has been improved. Since rapamycin or a derivative thereof is extremely instable to oxygen, light and moisture, it is problematic in terms of preservation stability. The present invention is a technique relating to a pharmaceutical formulation composition, in which the preservation stability of such a compound has been improved by a stabilizer having excellent safety with consideration to *in vivo* administration.

Background Art

It has been known that rapamycin (sirolimus) is a macrolide antibiotic discovered from the metabolite of actinomyces and has an immunosuppressive action. Rapamycin has an action to inhibit a mammalian rapamycin target protein (mammalian target of rapamycin; mTOR) that regulates cell division, cell growth, survival, etc. This mTOR is a main serine-threonine kinase, which regulates the synthesis of proteins by stimulation with growth factors, nutrients, etc., and the mTOR has been known to regulate the growth, proliferation and survival of cells, and angiogenesis. Hence, the mTOR inhibitory action of rapamycin has been focused, and the synthesis of a derivative thereof has been attempted. As a result, everolimus and temsirolimus have been discovered as antitumor agents.

A pharmaceutical formulation used to provide a pharmaceutical product comprising rapamycin or a derivative thereof has been reported. Patent Literature 1 discloses that a solution containing a mixture of rapamycin, hydroxypropylmethyl cellulose, lactose and the like is prepared, the solvent is then distilled away from the solution, and the obtained solid dispersion is then formulated. In addition, Patent Literature 2 discloses a tablet comprising everolimus used as a rapamycin, crospovidone used as a disintegrator, colloidal silicon dioxide, and lactose.

Rapamycin or a derivative thereof has been known to have physical properties by which it is extremely instable to oxidation. Hence, in general, an antioxidant is added to a pharmaceutical product formulation comprising, as an active ingredient, rapamycin. For instance, to a rapamycin preparation (registered trademark: Rapalimus) and a temsirolimus preparation (registered trademark: Torisel), tocopherol is added. In addition, for everolimus formulations (registered trademark: Afinitor and Certican), a synthetic antioxidant, dibutylhydroxytoluene (BHT) is used.

Regarding a method of stabilizing a rapamycin derivative using an antioxidant, Patent Literature 3 discloses that a mixed solution containing everolimus and BHT as an antioxidant is prepared, and the solvent is then removed from the mixed solution, so as to obtain a stabilized everolimus solid. Examples of the antioxidant used in this publication include BHT, tocopherol and ascorbic acid.

However, it has been reported that BHT used as an antioxidant exhibits carcinogenicity or reproduction toxicity, and thus, this is a chemical substance, the amount used of which is limited. On the other hand, tocopherol and ascorbic acid have higher safety than BHT, but its antioxidative activity is lower than that of BHT. Thus, the effect of tocopherol and ascorbic acid to stabilize rapamycin based on its antioxidative action has a certain limit.

As other antioxidants used as additives for pharmaceutical products, there have been known ascorbyl palmitate ester and ascorbyl stearate ester as a fat-soluble derivative of ascorbic acid. Further, it has been known that citric acid or ethylenediaminetetraacetic acid (EDTA) as a chelating agent is applied. However, when such substance is applied to a formulation comprising rapamycin or a derivative thereof, the effect of suppressing the decompsotion of rapamycin or a derivative thereof is not sufficient, and the decompsotion may be promoted in some cases.

Patent Literature 4 discloses that an ethanol solution of everolimus is added to a water-soluble polymer such as hypromellose, and the mixture is then granulated to prepare a solid dispersion, thereby obtaining a stable everolimus composition, without using antioxidants.

Prior Art Literatures

Patent Literatures

Patent Literature 1: JP Patent Publication (Kohyo) No. 11-509223 A (1999)
Patent Literature 2: JP Patent Publication (Kohyo) No. 2005-507897 A
Patent Literature 3: JP Patent Publication (Kohyo) No. 2002-531527 A
Patent Literature 4: International Publication No. WO 2013/022201

Summary of Invention

Object to be Solved by the Invention

It is an object of the present invention to provide a pharmaceutical composition and a pharmaceutical formulation, used in a pharmaceutical formulation comprising rapamycin or a derivative thereof, wherein the pharmaceutical composition can suppress a reduction in the content of an active ingredient caused by oxidation or decomposition of the rapamycin or a derivative thereof, can ensure long-term stability, and has high safety. It is another object of the present invention to provide a method for producing a pharmaceutical composition and a pharmaceutical formulation comprising rapamycin or a derivative thereof, wherein the composition and formulation can ensure long-term stability and has high safety.

Means for Solving the Object

The present inventors have found that, the decomposition of rapamycin or a derivative thereof in a pharmaceutical formulation comprising the rapamycin or a derivative thereof, can be stabilized for a long period of time by using antioxidant compounds used therein at optimum pH range. Namely, the stabilization effect on rapamycin or a derivative thereof could be successfully maintained for a long period of time by preparing a suitable salt of ascorbic acid and/or chelating agent as antioxidant compound and applying it. Specifically, the present application includes the inventions according to the following [1] to [9] as features.

[1] A pharmaceutical composition comprising (A) rapamycin or a derivative thereof, and (B) a salt of an ascorbic acid or a derivative thereof and/or a salt of chelating agent.
In the present invention, a pharmaceutical composition is allowed to comprise (B) a salt of an ascorbic acid or a derivative thereof and/or a salt of chelating agent with respect to the rapamycin or a derivative thereof, so that the composition, in which the stability of the rapamycin or a derivative thereof is improved, can be produced.
[2] The pharmaceutical composition according to the above [1], which is produced by preparing a solution containing (A) rapamycin or a derivative thereof, and (B) a salt of an ascorbic acid or a derivative thereof and/or a salt of chelating agent, and then removing the solvent from the solution.
As an aspect of mixing the rapamycin or a derivative thereof with (B) a salt of an ascorbic acid or a derivative thereof and/or a salt of chelating agent, it is preferable to prepare a solution containing the above-described components (A) and (B), and then to obtain a solid mixture from the solution. Such a solid mixture is a mixture formed by associating the rapamycin or a derivative thereof with the salt of an ascorbic acid or a derivative thereof and/or the salt of chelating agent at a molecular level, and thus, it is difficult to explain this solid mixture with a chemical structure, properties, etc. Hence, a more detailed aspect of the pharmaceutical composition according to the present invention, which comprises (A) rapamycin or a derivative thereof and (B) a salt of an ascorbic acid or a derivative thereof and/or a salt of chelating agent, is appropriately expressed as a solid mixture comprising rapamycin or a derivative thereof, and a salt of an ascorbic acid or a derivative thereof and/or a salt of chelating agent, which is specified by the production method according to the above [2], and this pharmaceutical composition is considered to satisfy the requirements regarding the clarity of the invention.
[3] The pharmaceutical composition according to the above [1] or [2], wherein (B) the salt of an ascorbic acid or the derivative thereof and/or a salt of chelating agent has a pH of 4 to 11 in 5mg/mL 50%(v/v) ethanol aqueous solution.
[4] The pharmaceutical composition according to any one of the above [1] to [3], which comprises 0.0001 to 20.0 parts by mass of (B) a salt of an ascorbic acid or a derivative thereof and/or a salt of chelating agent based on 1 part by mass of (A) the rapamycin or a derivative thereof.
[5] The pharmaceutical composition according to any one of the above [1] to [4], which comprises a cellulose derivative and/or sugars.
[6] A pharmaceutical formulation comprising the pharmaceutical composition according to any one of the above [1] to [5].
Moreover, the present invention also includes, as a feature thereof, a method for producing a pharmaceutical composition comprising rapamycin or a derivative thereof, and a salt of an ascorbic acid or a derivative thereof and/or a salt of chelating agent.
[7] A method for producing a pharmaceutical composition comprising rapamycin or a derivative thereof, which comprises preparing a solution containing (A) rapamycin or a derivative thereof, and (B) a salt of an ascorbic acid or a derivative thereof and/or a salt of chelating agent, and then removing the solvent from the solution.
According to the above-described production method, a solid mixture, in which rapamycin or a derivative thereof is associated with a salt of an ascorbic acid or a derivative thereof and/or a salt of chelating agent at a molecular level, can be produced. Furthermore, when an antioxidant other than (B) the salt of an ascorbic acid or a derivative thereof and/or the salt of chelating agent, such an antioxidant may be added upon preparation of the solution, or may also be added to the solid mixture.

[8] A method for producing a pharmaceutical formulation comprising rapamycin or a derivative thereof, which comprises preparing a solution containing (A) rapamycin or a derivative thereof, and (B) a salt of an ascorbic acid or a derivative thereof and/or a salt of chelating agent, then mixing the solution with a cellulose derivative and/or sugars, and then removing the solvent from the mixture.

[9] A method for producing a pharmaceutical formulation comprising rapamycin or a derivative thereof, which comprises preparing a solution containing (A) rapamycin or a derivative thereof, and (B) a salt of an ascorbic acid or a derivative thereof and/or a salt of chelating agent, and then removing the solvent from the prepared solution to obtain a pharmaceutical composition comprising the rapamycin or a derivative thereof, and adding a cellulose derivative and/or sugars to the above-described pharmaceutical composition.

As in the production method according to the above [8] or [9], upon production of a pharmaceutical formulation comprising rapamycin or a derivative thereof, the cellulose derivative and/or sugars used as pharmaceutical additives may be added at the stage of preparing a solid mixture, in which the rapamycin or a derivative thereof is associated with the salt of an ascorbic acid or a derivative thereof and/or the salt of chelating agent at a molecular level, or may also be added after preparation of the solid mixture.

Advantageous Effects of Invention

According to the present invention, there can be provided a pharmaceutical composition comprising rapamycin or a derivative thereof, wherein the pharmaceutical composition can suppress a reduction in the content of an active ingredient caused by oxidation or decomposition of the rapamycin or a derivative thereof, can ensure long-term stability, and has high safety. That is to say, long-term stability of rapamycin or a derivative thereof can be maintained for a long period of time by preparing a suitable salt of acidic antioxidant such as an ascorbic acid or a derivative thereof or chelating agent (such as citric acid or EDTA) having an optimum pH range and using it as an antioxidant. Further, the pharmaceutical composition and pharmaceutical formulation of the present invention are pharmaceutical formulations, which use additives having assured safety, and avoid the use of BHT that is problematic in terms of carcinogenicity or reproduction toxicity. Pharmaceutical formulations comprising rapamycin or a derivative thereof which ensure stability and safety, can be provided.

Embodiments for Carrying out the Invention

The pharmaceutical composition of the present invention is characterized in that it comprises (A) rapamycin or a derivative thereof, and (B) a salt of an ascorbic acid or a derivative thereof and/or a salt of chelating agent. The details thereof will be described below.

The present invention comprises, as an active ingredient, (A) rapamycin or a derivative thereof.

Rapamycin (common name: Sirolimus) is a compound having a macrolide skeleton that has been isolated from the metabolite of actinomyces, *Streptomyces Hygroscopicus,* separated from the soil of Easter Island.

The term "rapamycin derivative" means a substance prepared by chemically modifying rapamycin used as a mother core. Examples of the rapamycin derivative include 16-O-substituted rapamycin (see, for example, WO 94/022136), 40-O-substituted rapamycin (see, for example, US 5258389 and WO 94/09010), carboxylic acid ester-substituted rapamycin (see, for example, WO 92/05179), amide-substituted rapamycin (see, for example, US 5118677), fluorine-substituted rapamycin (see, for example, US 5100883), and acetal-substituted rapamycin (see, for example, US 5151413). The rapamycin or a derivative thereof of the present invention is not limited thereto, but the aforementioned rapamycin derivatives can be used as applicable preferred compounds.

The rapamycin derivative is preferably a 40-O-substituted rapamycin derivative, in which the hydroxyl group at position 40 of the cyclohexyl group of rapamycin is substituted with a hydroxyalkyl group, a hydroxyalkoxyalkyl group, an acylaminoalkyl group, an aminoalkyl group, or a hydroxy-substituted acyl group. The rapamycin derivative is more preferably 40-O-(2-hydroxyethyl)rapamycin (everolimus), or 40-O-[3-hydroxy-2-(hydroxymethyl)-2-methylpropanoate]rapamycin (temsirolimus).

As (A) rapamycin or a derivative thereof of the present invention, rapamycin (Sirolimus), everolimus, or temsirolimus is preferably used.

As such (A) rapamycin or a derivative thereof, a compound having a quality that can be sufficiently used as a pharmaceutical product is preferably used.

Ascorbic acid in (B) the salt of an ascorbic acid or a derivative thereof and/or the salt of chelating agent used in the present invention is a compound which is referred to as (R)-3,4-dihydroxy-5-((S)-1,2-dihydroxyethyl)furan-2(5H)one as chemical name. It is known as vitamin C, and widely and universally exists in nature, for example in fruits such as lemon, grapefruit, kiwifruit and strawberry and vegetables such as spinach and potato.

As the derivative of ascorbic acid includes ascorbic acid fatty acid ester wherein fatty acid is bound via ester-bond to ascorbic acid or erythorbic acid which is a stereoisomer of ascorbic acid. The ascorbic acid fatty acid ester is not particularly limited and any compound can be used herein, as long as it has a structure formed by introducing fatty acid into ascorbic acid via an ester bond.

An example of the fatty acid to be bound to ascorbic acid via an ester bond is monocarboxylic acid containing 2 to 30 carbon atoms (C2-C30). The fatty acid may be saturated fatty acid, or may also be unsaturated fatty acid containing one or more double bonds. Examples of the saturated fatty acid include acetic acid, propionic acid, butanoic acid, pentanoic acid, hexanoic acid, palmitic acid, stearic acid, eicosanoic acid, and docosanoic acid.

Examples of the unsaturated fatty acid include crotonic acid, myristoleic acid, palmitoleic acid, oleic acid, linoleic acid, and linolenic acid.

Examples of the ascorbic acid fatty acid ester are preferably those used as pharmaceutical additive and food additive, and include L-ascorbyl stearate and ascorbyl palmitate.

In the present invention, an ascorbic acid or a derivative thereof in connection with (B) may be each used alone, or may also be used in combination.

Examples of (B) a salt of an ascorbic acid or a derivative thereof include inorganic salts such as a salt with sodium or potassium, and amine salts such as a salt with ammonia, diethanolamine, monoethanolamine, triethanolamine, and Meglumine.

(B) A salt of an ascorbic acid or a derivative thereof may be used in such a way that a salt is formed in the pharmaceutical composition of the present invention by allowing an ascorbic acid or a derivative thereof to coexist with conjugate base component. The conjugate base is not particularly limited so long as it shows alkaline property when an aqueous solution is prepared. When inorganic salt is prepared, hydroxide such as sodium hydroxide or potassium hydroxide; carbonate and the like such as sodium carbonate, potassium carbonate, sodium bicarbonate or potassium bicarbonate; acetate such as sodium acetate or potassium acetate; or phosphate such as sodium phosphate or potassium phosphate; and the like may be added. When amine salt is prepared, amine compound such as ammonia, diethanolamine, monoethanolamine, triethanolamine, and Meglumine, may be used.

(B) The salt of an ascorbic acid or a derivative thereof is preferably an alkaline metal salt such as sodium salt or potassium salt, and/or amine salt such as ammonium salt or Meglumine salt. As mentioned above, these salts may be used in such a way that an ascorbic acid or a derivative thereof is allowed to coexist with compound comprising conjugate base component such as alkaline metal hydroxide, carbonate or amine.

In the present invention, a salt of chelating agent may be used as the (B) component. Examples of the chelating agent include acidic chelating agent which is carboxylic acid derivative such as citric acid, ethylenediaminetetraacetic acid (EDTA) or gluconic acid. The chelating agent is known to have an antioxidant action which traps metal ion and suppresses the oxidation reaction which is catalyzed by metal. In the present invention, citric acid or EDTA is preferably used as a chelating agent. More preferably, citric acid is used.

Examples of a salt of chelating agent include inorganic salts such as a salt with sodium or potassium, and amine salts such as a salt with ammonia, diethanolamine, monoethanolamine, triethanolamine, and Meglumine.

(B) The salt of chelating agent may be used in such a way that a salt is formed in the pharmaceutical composition of the present invention by adding conjugate base component to the chelating agent. The conjugate base is not particularly limited so long as it shows alkaline property when an aqueous solution is prepared. When inorganic salt is prepared, hydroxide such as sodium hydroxide or potassium hydroxide; carbonate and the like such as sodium carbonate, potassium carbonate, sodium bicarbonate or potassium bicarbonate; acetate such as sodium acetate or potassium acetate; or phosphate such as sodium phosphate or potassium phosphate; and the like may be added. When amine salt is prepared, amine compound such as ammonia, diethanolamine, monoethanolamine, triethanolamine, and Meglumine, may be used.

(B) The salt of chelating agent is preferably an alkaline metal salt such as sodium salt or potassium salt, and/or amine salt such as ammonium salt or Meglumine salt. As mentioned above, these salts may be used in such a way that a chelating agent is allowed to coexist with compound comprising conjugate base component such as alkaline metal hydroxide, carbonate or amine.

As (B) the salt of an ascorbic acid or the derivative thereof and/or a salt of chelating agent in the present invention, a salt having a pH of 4 to 11 in 5mg/mL 50%(v/v) ethanol aqueous solution is preferably used. Namely, in order to suppress the decomposition of rapamycin or a derivative thereof and maintain the stability for a long period of time, ascorbic acid or chelating agent having an antioxidant action is used at optimum pH range. Preferably, the pH range under the aforementioned measurement condition is 5 to 10.

The pH of the (B) component can be measured by preparing 50%(v/v) ethanol aqueous solution containing 5mg/mL of the (B) component, and measuring pH using commercially available pH meter. For example, pH can be measured by using MM-60R (TOA DKK).

In order to prepare a salt of an ascorbic acid or the derivative thereof and/or a salt of chelating agent having a pH of 4 to 11 in 5mg/mL 50%(v/v) ethanol aqueous solution as the (B) component, a basic component may be added to an ascorbic acid or the derivative thereof or chelating agent which is acidic, so that pH is adjusted. Therefore, in order to prepare a salt having pH of 4 to 11, preferably pH of 5 to 10, the pH can be adjusted by a method of adjusting the basic degree of the basic component and/or a method of adjusting the amount of the basic component. Namely, when sodium ascorbate is used as the (B) component, it is preferably used in such a way that sodium ascorbate or ascorbic acid and sodium ascorbate are coexisting. When sodium citrate is used as the (B) component, one of monosodium citrate, diosodium citrate and trisodium citrate, or mixture thereof is preferably used.

In the present invention, (B) the salt of an ascorbic acid or the derivative thereof and/or a salt of chelating agent may be used in an amount of preferably 0.001 part by mass or more, more preferably 0.005 parts by mass or more, and further preferably 0.01 part by mass or more, based on 1 part by mass of (A) the rapamycin or a derivative thereof. In the present invention, since the salt of an ascorbic acid or the derivative thereof and/or a salt of chelating agent does not have physical properties that impair the stability of the rapamycin or a derivative thereof, the upper limit of the use amount thereof is not particularly limited, and should be determined, as appropriate, in an amount practically usable as a pharmaceutical product.

Taking into consideration the ensuring of the stability of the rapamycin or a derivative thereof and the realistic use amount of a pharmaceutical product additive, the salt of an ascorbic acid or the derivative thereof and/or a salt of citric acid may be used in an amount of preferably 0.001 to 20 parts by mass, more preferably 0.001 to 10 parts by mass, and further preferably 0.005 to 1.0 part by mass, based on 1 part by mass of (A) the rapamycin or a derivative thereof.

In the present invention, as the (B) component, the salt of an ascorbic acid or the derivative or the salt of chelating agent may be used respectively alone, or two or more (B) components may be used in combination since the mechanism of antioxidant action of ascorbic acid and the like is different from that of chelating agent. When two or more (B) components are used in combination, the mixing ratio can be appropriately set so that the stability of rapamycin or a derivative thereof is ensured.

The pharmaceutical composition of the present invention comprises at least two components, namely, (A) rapamycin or a derivative thereof, and (B) a salt of an ascorbic acid or the derivative thereof and/or a salt of chelating agent, and an aspect of mixing these components is not particularly limited and any mixing aspect is included in the present invention, as long as it is an aspect in which the components are mixed and are thus present. As a method of mixing these components, a method, which comprises mixing (A) the rapamycin or a derivative thereof and (B) a salt of an ascorbic acid or the derivative thereof and/or a salt of chelating agent in the state of a solid and mechanically blending them using any given mixer or the like, is applied. During such mixing operation, a suitable solvent may be added to the components, so that a dispersion of the components may be promoted. When the components (A) and (B) to be mixed are all solids, powdery or granular components having a small mean particle diameter of approximately 0.1 to 1 mm are advantageously used in terms of the improvement of dispersibility.

In the pharmaceutical composition of the present invention, the aspect, in which the components, namely, (A) the rapamycin or a derivative thereof and (B) a salt of an ascorbic acid or the derivative thereof and/or a salt of chelating agent are mixed and are thus present, is preferably a solid mixture comprising (A) the rapamycin or a derivative thereof and (B) a salt of an ascorbic acid or the derivative thereof and/or a salt of chelating agent, which is obtained by preparing a solution containing these components and then removing the solvent from the solution. Such a mixture is considered to a mixture, in which the rapamycin or a derivative thereof is associated with the salt of an ascorbic acid or the derivative thereof and/or the salt of chelating agent at a molecular level, and thus, this is a mixing form capable of exhibiting the highest stabilization effects of (B) the salt of an ascorbic acid or the derivative thereof and/or the salt of chelating agent on the rapamycin or a derivative thereof.

The solvent that can be used to prepare a solution containing (A) rapamycin or a derivative thereof and (B) a salt of an ascorbic acid or the derivative thereof and/or a salt of chelating agent is not limited, as long as both (A) rapamycin or a derivative thereof and (B) a salt of an ascorbic acid or the derivative thereof and/or a salt of chelating agent are dissolved therein. Examples of such a solvent include water, methanol, ethanol, 1-propanol, 2-propanol, 1-butanol, 2-butanol, 2-methyl-1-propanol, 3-methyl-1-butanol, 1-pentanol, ethylene glycol, glycerin, formic acid, acetic acid, acetone, methyl ethyl ketone, methyl isobutyl ketone, anisole, methyl acetate, ethyl acetate, ethyl formate, propyl acetate, isopropyl acetate, n-butyl acetate, isobutyl acetate, acetonitrile, diethyl ether, t-butyl methyl ether, tetrahydrofuran, 1,4-dioxane, diisopropyl ether, pentane, hexane, heptane, N,N-dimethylformamide, N,N-dimethylacetamide, N-methylpyrrolidone, 1,3-dimethyl-2-imidazolidinone, dimethyl sulfoxide, dichloromethane, and chloroform. These solvents may be used alone, or may also be used as a mixed solvent comprising two or more types of solvents. Examples of the solvent used herein are not limited thereto, but the aforementioned solvents can be used as applicable preferred solvents.

Taking into consideration the subsequent removal of a solvent, it is preferable to use a solvent having a boiling point of 120°C or lower, which can be distilled away under mild conditions. Examples of such a preferred solvent include water, methanol, ethanol, propanol, acetonitrile, acetone, methyl ethyl ketone, ethyl acetate, butyl acetate, tetrahydrofuran, 1,4-dioxane, pentane, heptane, diethyl ether, and t-butyl methyl ether.

The amount of the solvent used is not particularly limited, as long as (A) rapamycin or a derivative thereof and (B) the salt of an ascorbic acid or the derivative thereof and/or the salt of chelating agent are completely dissolved in the solvent. Hence, the amount of the solvent used can be adjusted, as appropriate.

In addition, upon preparation of the solution, the temperature is increased, as appropriate, so that dissolution of (A) rapamycin or a derivative thereof and (B) the salt of an ascorbic acid or the derivative thereof and/or the salt of chelating agent may be promoted. The temperature of the solution upon preparation thereof is not particularly limited. Taking into consideration the stability of (A) rapamycin or a derivative thereof, it is preferable to prepare the solution at a temperature of 0°C to 80°C.

The method of preparing a solution containing rapamycin or a derivative thereof, and a salt of an ascorbic acid or the derivative thereof and/or a salt of chelating agent is not particularly limited in the present invention, as long as it is a method of dissolving the rapamycin or a derivative thereof and the salt of an ascorbic acid or the derivative thereof and/or the salt of chelating agent in the solution. Examples of such a method include: a method, which comprises previously mixing rapamycin or a derivative thereof and (B) a salt of an ascorbic acid or the derivative thereof and/or a salt of chelating agent, then adding a solvent, in which the components are dissolved, to the mixture, and then dissolving the components in the solvent; and a method, which comprises mixing a solution prepared by adding a solvent to rapamycin or a derivative, with a solution prepared by adding a solvent to a salt of an ascorbic acid or the derivative thereof and/or a salt of chelating agent. The method of preparing a solution containing rapamycin or a derivative thereof and a salt of an ascorbic acid or the derivative thereof and/or a salt of chelating agent is not limited thereto, but these preparation methods can be used as applicable preferred preparation methods.

As a method of removing a solvent from a solution containing (A) rapamycin or a derivative thereof and (B) the salt of an ascorbic acid or the derivative thereof and/or the salt of chelating agent, a method of distilling the solvent away from the solution is applied. Regarding such a method of distilling the solvent away from the solution, the solvent can be removed from the solution by heating the solution. At that time, reduced pressure conditions are preferably applied because the solvent can be removed under mild temperature conditions. Otherwise, the solvent can also be removed by a spray-drying method, so as to obtain a solid mixture.

Moreover, there may also be adopted a method, which comprises precipitating a solid mixture comprising (A) rapamycin or a derivative thereof and (B) the salt of an ascorbic acid or the derivative thereof and/or the salt of chelating agent from a solution containing the components (A) and (B), and then removing the solvent according to a filtration method. Examples of the method of precipitating a solid mixture include: what is called, a recrystallization method of promoting crystallization by cooling; and what is called, a precipitation method of adding a solvent for crystallization, which is miscible with a solution containing (A) rapamycin or a derivative thereof and (B) the salt of an ascorbic acid or the derivative thereof and/or the salt of chelating agent, and in which the components (A) and (B) are insoluble or hardly-soluble, to the solid mixture, followed by crystallization.

The above-described solvent for crystallization is not particularly limited and any solvent can be applied herein, as long as it is miscible with a solution containing (A) rapamycin or a derivative thereof and (B) the salt of an ascorbic acid or the derivative thereof and/or the salt of chelating agent, and also, the components (A) and (B) are insoluble or hardly soluble therein. Examples of the solvent for crystallization include water, diethyl ether, diisopropyl ether, tetrahydrofuran, 1,4-dioxane, hexane, heptane, and toluene. The type and use amount of the solvent for crystallization may be determined, as appropriate, depending on the type and amount of a solvent used in preparation of the solution.

A solvent for crystallization is added to a solution containing (A) rapamycin or a derivative thereof and (B) the salt of an ascorbic acid or the derivative thereof and/or the salt of chelating agent, so that a solid mixture containing the components (A) and (B) is crystallized. After that, the mixture is arbitrarily cooled to promote crystallization, so as to prepare a suspension. Thereafter, the solvent is removed from the suspension by a filtration method, so as to obtain a solid mixture.

In the pharmaceutical composition of the present invention, as a stabilization adjuvant for ensuring the stability of the rapamycin or a derivative thereof, an antioxidant other than (B) the salt of an ascorbic acid or the derivative thereof and/or the salt of chelating agent can be arbitrarily combined with (B), and can be then used.

As such an antioxidant, a known antioxidant exhibiting the effect of stabilizing the rapamycin and a derivative thereof can be used. Examples of such a known antioxidant include tocopherol, nitrite, sulfite, alpha-thioglycerin, cysteine hydrochloride, dichloroisocyanuric acid, dibutylhydroxytoluene, lecithin, thioglycolic acid, thiomalic acid, pyrosulfite, butylhydroxyanisole, 1,3-butylene glycol, pentaerythritol tetrakis[3-(3,5-di-t-butyl-4-hydroxyphenyl)propionate]benzotriazole, isopropyl gallate, and 2-mercaptobenzimidazole. Examples of the antioxidant used herein are not limited thereto, but these compounds can be used as applicable preferred compounds.

The antioxidant can be used, as appropriate, in an amount that does not impair the stability of the rapamycin or a derivative thereof. When the other antioxidant is used, the addition amount is not particularly limited. The antioxidant is preferably used in an amount of 0.0001 to 10 parts by mass, based on 1 part by mass of (A) the rapamycin or a derivative thereof. The antioxidant is used in an amount of more preferably 0.001 to 1.0 parts by mass, and further preferably 0.005 to 1.0 parts by mass.

The above-described other antioxidant is used by being added, separately, to a mixture of (A) the rapamycin or a derivative thereof and (B) the salt of an ascorbic acid or the derivative thereof and/or the salt of chelating agent.

Alternatively, the antioxidant may be used by being added to a solid mixture prepared from the solution of the components (A) and (B). Otherwise, a solution containing the components (A) and (B) and an antioxidant is prepared, and the solvent is then removed from the solution, so that the antioxidant may be used in the form of a solid mixture comprising the components (A) and (B) and the antioxidant.

When the antioxidant is used in the pharmaceutical composition of the present invention, it is preferable that a solution containing (A) the rapamycin or a derivative thereof, (B) the salt of an ascorbic acid or the derivative thereof and/or the salt of chelating agent, and the other antioxidant be prepared, the solvent be removed from the solution, and the antioxidant be used in the form of a solid mixture comprising the components (A) and (B) and the antioxidant. Examples of the method of removing the solvent, which is applied in the method of obtaining such a solid mixture, include a method involving distillation of the solvent and a method involving crystallization and filtration, as with the aforementioned methods. As such a solvent distillation method, a spray-drying method may also be used.

To the pharmaceutical composition of the present invention, a cellulose derivative and/or sugars may be added. These substances are used as pharmaceutical additives for preparing pharmaceutical product formulations.

The cellulose derivative is not particularly limited and any cellulose derivative can be used herein, as long as it is an additive that is commonly used in preparation of pharmaceutical product formulations. Examples of the cellulose derivative include crystalline cellulose, methyl cellulose, ethyl cellulose, cellulose acetate phthalate, low-substituted hydroxypropyl cellulose, hydroxypropyl cellulose, hydroxymethylpropyl cellulose, hydroxypropylmethyl cellulose acetate succinate, and hydroxypropylmethyl cellulose phthalate. Among these, it is preferable to use crystalline cellulose, methyl cellulose, ethyl cellulose, low-substituted hydroxypropyl cellulose, hydroxypropyl cellulose, and hydroxypropylmethyl cellulose.

Moreover, the sugars are not particularly limited and any sugars can be used herein, as long as they are additives that are commonly used in preparation of pharmaceutical product formulations. Examples of the sugars include arabinose, isomaltose, inositol, erythritol, galactosamine, galactose, xylitol, xylose, glucosamine, glucose, gentiobiose, kojibiose, sucrose, cellobiose, sophorose, sorbitol, thioglucose, turanose, deoxyribose, nigerose, palatinose, fucose, fructose, mannitol, maltose, mannose, melibiose, lactose, rhamnose, laminaribiose and trehalose. Among these, it is preferable to use lactose, mannitol, maltose, erythritol, sorbitol, fucose, xylitol, fructose, inositol, and trehalose.

The above-described cellulose derivative and/or sugars may be used alone, or in combination of multiple types.

The cellulose derivative and/or the sugars are used by being added to a pharmaceutical composition comprising (A) the rapamycin or a derivative thereof and (B) the salt of an ascorbic acid or the derivative thereof and/or the salt of chelating agent, and optionally given other antioxidant.

That is to say, the cellulose derivative and/or the sugars are used by being added to (A) the rapamycin or a derivative thereof and (B) the salt of an ascorbic acid or the derivative thereof and/or the salt of chelating agent, and optionally given other antioxidant, so as to prepare a mixture (addition method 1). Alternatively, the cellulose derivative and/or the sugars may be added to a solid mixture prepared from a solution containing the components (A) and (B) and optionally given other antioxidant, so as to prepare a mixture (addition method 2).

When the cellulose derivative and/or sugars that are in a solid state are mixed with the components (A) and (B) and optionally given other antioxidant that are also in a solid state, it is preferable that these components be mechanically mixed with one another using a mixer or the like, so that they are fully dispersed.

Furthermore, as an alternative method, a solution containing the components (A) and (B) and optionally given antioxidant is mixed with the cellulose derivative and/or the sugars, and the solvent is then removed from this mixture, so that a mixture consisting of a solid mixture of the components (A) and (B) and optionally given other antioxidant, and the cellulose derivative and/or the sugars, may be obtained and used (addition method 3).

The above-described addition method 3 is a method of adding the cellulose derivative and/or the sugars to a solution containing the components (A) and (B) and optionally given other antioxidant. In this case, the cellulose derivative and/or the sugars are not necessarily dissolved in the solution, and they may be in the state of a suspension. The solvent is removed from this mixture, so that a mixture consisting of a solid mixture of the components (A) and (B) and optionally given antioxidant, and the cellulose derivative and/or the sugars, can be prepared. As a method of removing the solvent, a method of distilling away the solvent is applied. The solvent is preferably distilled away under reduced pressure. Otherwise, a method of removing the solvent according to a spray-drying method may also be adopted.

The above-described solvent for crystallization is added to the solution to prepare a suspension, and the solvent is then removed by filtration, so that a mixture consisting of a solid mixture comprising the components (A) and (B) and optionally given other antioxidant, and the cellulose derivative and/or the sugars, can be prepared.

In addition to the aforementioned salt of an ascorbic acid or the derivative thereof and/or salt of chelating agent, as well as optionally given other antioxidant, cellulose derivative and sugars, the pharmaceutical composition of the present invention may also comprise other additives that are commonly used in preparation of pharmaceutical product formulations, in a range that does not impair the effects of the present invention. The present pharmaceutical composition may comprise, for example, an excipient, a disintegrator, a binder, a lubricant, a pH adjuster, inorganic salts, and a solvent.

Examples of the excipient include lactose, maltose, mannitol, erythritol, sorbitol, fucose, xylitol, fructose, inositol, and starch.

Examples of the disintegrator include carmellose, crospovidone, low-substituted hydroxypropyl cellulose, sodium starch glycolate, carmellose calcium, and croscarmellose sodium.

Examples of the binder include hydroxypropyl cellulose, hypromellose, polyvinyl alcohol, and polyvinyl pyrrolidone.

Examples of the lubricant include magnesium stearate, calcium stearate, sodium stearyl fumarate, talc, and sucrose fatty acid ester.

Examples of the pH adjuster include hydrochloric acid, sulfuric acid, phosphoric acid, citric acid, tartaric acid, malic acid, mesylic acid, tosylic acid, and besylic acid. A buffer, which comprises, as a main ingredient, such an acidic additive, and further, an alkaline metal salt, an alkaline-earth metal salt, or an ammonium salt, may also be used.

Examples of the inorganic salts include calcium chloride, sodium chloride, calcium oxide, and magnesium sulfate.

In general, examples of the solvent include water, a normal saline, a 5% glucose or mannitol aqueous solution, a water-soluble organic solvent (e.g., a single solvent such as glycerol, ethanol, dimethyl sulfoxide, N-methylpyrrolidone, polyethylene glycol, Cremophor, or a mixed solvent thereof), and polyethylene glycols (e.g., polyethylene glycol 300, polyethylene glycol 400, polyethylene glycol 600, polyethylene glycol 4000, etc.).

These additives can be used without any particular limitation, as long as they have purity that is acceptable for the intended use as pharmaceutical product formulations. These additives may be used alone, or may also be used as a mixture of the additives. These additives are optionally used, when the pharmaceutical composition or the pharmaceutical formulation is produced.

The pharmaceutical composition of the present invention can be produced in the form of a pharmaceutical product comprising the pharmaceutical composition.

Examples of the dosage form of this pharmaceutical product include: internal use agents, such as a tablet, a dispersible tablet, a chewable tablet, an effervescent tablet, a troche, a drop agent, a hard capsule, a soft capsule, a granule, a powder agent, a pill, dry syrup, infusions and/or decoctions, an electuary, syrup, a drink agent, a suspension, an orally disintegrating tablet, and a jelly agent; and external use agents, such as a suppository, a poultice, a plaster, an ointment, a cream agent, a mousse agent solution, a liquid agent, eye drops, an aerosol agent, and a spray agent. The dosage forms are not limited thereto, but these are applicable preferred dosage forms.

In a case where the pharmaceutical composition of the present invention is used in the form of an injection, examples of such an injection include an aqueous injection, a non-aqueous injection, a suspension injection, an emulsion injection, and also, as dosage forms of being dissolved or suspended at the time of use, an intradermal injection, a subcutaneous injection; an intramuscular injection, an intravenous injection, a central intravenous injection, an intra-arterial injection, and an intrathecal injection. Examples of the injection used herein are not limited thereto, but these injections can be used as applicable preferred dosage forms and administration routes.

The pharmaceutical product, in which the pharmaceutical composition of the present invention is used, can be applied to the treatment of disease. Examples of the disease, to which the pharmaceutical product can be applied include: transplant rejection in transplantation of heart, lung, combined heart-lung, liver, kidney, pancreas, skin, or cornea; autoimmune diseases, and inflammatory diseases, such as arthritis, rheumatic disease, systemic lupus erythematosus, multi polychondritis, crusts disease, Wegener's granulomatosis, dermatomyositis, chronic active hepatitis, myasthenia gravis, psoriasis, Steven-Johnson syndrome, idiopathic sprue, autoimmune inflammatory bowel disease, endocrine ophthalmopathy, Graves disease, nodule colitis, multiple sclerosis, primary biliary hepatitis, juvenile diabetes (type 1 diabetes), uveitis, keratoconjunctivitis sicca, vernal keratoconjunctivitis, interstitial lung fibrosis, psoriatic arthritis, glomerulonephritis, and juvenile dermatomyositis; asthma; and cancers and hyperproliferative diseases, such as breast cancer, renal cancer, neuroendocrine tumor, lymphoid proliferative disease, B cell lymphatic cancer, tuberous sclerosis, and proliferative skin disease. Examples of the disease are not limited thereto, but these diseases can be considered to be applicable preferred diseases.

The applied dose of the pharmaceutical product comprising the pharmaceutical composition of the present invention can be naturally changed, depending on the sex, age, physiological conditions, pathologic conditions of a patient, etc. For example, the rapamycin or a derivative thereof is administered to an adult patient at a daily dose of 0.01 to 100 mg/m$^2$ (body surface area). The dose of the pharmaceutical product is not limited thereto, but this dose can be used as an applicable preferred dose.

Examples

Hereinafter, the present invention will be more specifically described in the following examples. However, these examples are not intended to limit the scope of the present invention.

It is to be noted that, in the analysis using liquid chromatography (HPLC) in the present test examples, the measurement was carried out under the following conditions.

Measurement column: Zorbax Eclipse XDB-C18, Rapid resolution HT, 100 mm x 4.6 mm, 1.8 $\mu$m
Detector: ultraviolet absorption spectrophotometer (measurement wavelength: 278 nm)
Column temperature: 45°C
Mobile phase A: 0.1% formic acid; mobile phase B: methanol/acetonitrile = 50/50 Concentration gradient of mobile phase:

[Table 1]

| Time after injection (min) | Mobile phase A (vol %) | Mobile phase B (vol %) |
|---|---|---|
| 0 - 5 | 46 | 64 |
| 5 - 17 | 46 → 25 | 64 → 75 |
| 17 - 22 | 25 → 10 | 75 → 90 |
| 22 - 24 | 10 | 90 |
| 24 - 25 | 10 → 46 | 90 → 64 |
| 25 - 28 | 46 | 64 |

Flow rate: 1.5 mL/min
Injection amount: 10 $\mu$L

[Example 1]

70 mg of Everolimus was weighed into a test tube, and 200 $\mu$L of anhydrous ethanol were then added thereto. The mixture was irradiated with ultrasonic wave for 10 minutes, and thereafter, dissolution of everolimus was confirmed. 10 $\mu$L of aqueous solution of sodium ascorbate (manufactured by FUSO CHEMICAL CO., LTD) (140 mg/mL) was then added thereto. Thereafter, 630 mg of anhydrous lactose (Super Tab (registered trademark) 21 AN, manufactured by DFE Pharma) and 70 mg of hypromellose (TC-5 E Type, manufactured by Shin-Etsu Chemical Co., Ltd.) were weighed into a mortar, and these substances were then stirred using a pestle to obtain a mixture. Thereafter, the above-obtained solution was added dropwise to this mixture, using a Pasteur pipette, and the thus obtained mixture was then stirred using a pestle. The obtained powders were transferred into an egg-plant shaped flask, and were then dried using an evaporator under reduced pressure for 3 hours, so as to produce the Everolimus-containing pharmaceutical composition of Example 1.
The pH of sodium ascorbate which was used as a stabilizer in Example 1 (at concentration of 5 mg/mL (in water/EtOH=1:1 mixture)) was measured by MM-60R (TOA DKK), and was found to be 8.4.

[Example 2]

70 mg of Everolimus was weighed into a test tube, and 200 $\mu$L of anhydrous ethanol were then added thereto. The mixture was irradiated with ultrasonic wave for 10 minutes, and thereafter, dissolution of everolimus was confirmed. 10 $\mu$L of aqueous solution of disodium hydrogen citrate (manufactured by Wako Pure Chemical Industries, Ltd) (140 mg/mL) was then added thereto. Thereafter, 630 mg of anhydrous lactose (Super Tab (registered trademark) 21 AN, manufactured by DFE Pharma) and 70 mg of hypromellose (TC-5 E Type, manufactured by Shin-Etsu Chemical Co., Ltd.) were weighed into a mortar, and these substances were then stirred using a pestle to obtain a mixture. Thereafter, the above-obtained solution was added dropwise to this mixture, using a Pasteur pipette, and the thus obtained mixture was then stirred using a pestle. The obtained powders were transferred into an egg-plant shaped flask, and were then dried using an evaporator under reduced pressure for 3 hours, so as to produce the Everolimus-containing pharmaceutical composition of Example 2.
The pH of disodium hydrogen citrate which was used as a stabilizer in Example 2 (at concentration of 5 mg/mL (in water/EtOH=1:1 mixture)) was measured by MM-60R (TOA DKK), and was found to be 6.3.

[Comparative Example 1]

70 mg of Everolimus was weighed into a test tube, and 100 $\mu$L of dibutylhydroxytoluene (BHT, manufactured by MERCK) anhydrous ethanol solution (14 mg/mL) was then added thereto. The mixture was irradiated with ultrasonic wave for 10 minutes, and thereafter, dissolution of everolimus was confirmed. After that, 100 $\mu$L of anhydrous ethanol was added to the mixed solution to dilute it. Thereafter, 630 mg of anhydrous lactose (Super Tab (registered trademark) 21 AN, manufactured by DFE Pharma) and 70 mg of hypromellose (TC-5 E Type, manufactured by Shin-Etsu Chemical Co., Ltd.) were weighed into a mortar, and these substances were then stirred using a pestle to obtain a mixture. Thereafter, the above-obtained solution was added dropwise to this mixture, using a Pasteur pipette, and the thus obtained mixture was then stirred using a pestle. The obtained powders were transferred into an egg-plant shaped flask, and were then dried using an evaporator under reduced pressure for 3 hours, so as to produce the Everolimus-containing pharmaceutical composition of Comparative Example 1.

The pH of BHT which was used as a stabilizer in Comparative Example 1 (at concentration of 2.5 mg/mL (in water/EtOH=1:3 mixture)) was measured by MM-60R (TOA DKK), and was found to be 8.6.

[Comparative Example 2]

70 mg of Everolimus was weighed into a test tube, and 100 μL of ascorbic acid (manufactured by FUSO CHEMICAL CO., LTD.) anhydrous ethanol solution (14 mg/mL) was then added thereto. The mixture was irradiated with ultrasonic wave for 10 minutes, and thereafter, dissolution of everolimus was confirmed. After that, 100 μL of anhydrous ethanol was added to the mixed solution to dilute it. Thereafter, 630 mg of anhydrous lactose (Super Tab (registered trademark) 21 AN, manufactured by DFE Pharma) and 70 mg of hypromellose (TC-5 E Type, manufactured by Shin-Etsu Chemical Co., Ltd.) were weighed into a mortar, and these substances were then stirred using a pestle to obtain a mixture. Thereafter, the above-obtained solution was added dropwise to this mixture, using a Pasteur pipette, and the thus obtained mixture was then stirred using a pestle. The obtained powders were transferred into an egg-plant shaped flask, and were then dried using an evaporator under reduced pressure for 3 hours, so as to produce the Everolimus-containing pharmaceutical composition of Comparative Example 2.

The pH of ascorbic acid which was used as a stabilizer in Comparative Example 2 (at concentration of 5 mg/mL (in water/EtOH=1:1 mixture)) was measured by MM-60R (TOA DKK), and was found to be 3.6.

[Comparative Example 3]

70 mg of Everolimus was weighed into a test tube, and 100 μL of citric acid (manufactured by Junsei Chemical Co., Ltd) anhydrous ethanol solution (14 mg/mL) was then added thereto. The mixture was irradiated with ultrasonic wave for 10 minutes, and thereafter, dissolution of everolimus was confirmed. After that, 100 μL of anhydrous ethanol was added to the mixed solution to dilute it. Thereafter, 630 mg of anhydrous lactose (Super Tab (registered trademark) 21 AN, manufactured by DFE Pharma) and 70 mg of hypromellose (TC-5 E Type, manufactured by Shin-Etsu Chemical Co., Ltd.) were weighed into a mortar, and these substances were then stirred using a pestle to obtain a mixture. Thereafter, the above-obtained solution was added dropwise to this mixture, using a Pasteur pipette, and the thus obtained mixture was then stirred using a pestle. The obtained powders were transferred into an egg-plant shaped flask, and were then dried using an evaporator under reduced pressure for 3 hours, so as to produce the Everolimus-containing pharmaceutical composition of Comparative Example 3.

The pH of citric acid which was used as a stabilizer in Comparative Example 3 (at concentration of 5 mg/mL (in water/EtOH=1: 1 mixture)) was measured by MM-60R (TOA DKK), and was found to be 3.5.

[Comparative Example 4]

70 mg of Everolimus was weighed into a test tube, and 100 μL of DL-α-tocopherol (Riken E Oil 1000, manufactured by RIKEN VITAMIN CO., LTD.) anhydrous ethanol solution (14 mg/mL) was then added thereto. The mixture was irradiated with ultrasonic wave for 10 minutes, and thereafter, dissolution of everolimus was confirmed. After that, 100 μL of anhydrous ethanol was added to the mixed solution to dilute it. Thereafter, 630 mg of anhydrous lactose (Super Tab (registered trademark) 21 AN, manufactured by DFE Pharma) and 70 mg of hypromellose (TC-5 E Type, manufactured by Shin-Etsu Chemical Co., Ltd.) were weighed into a mortar, and these substances were then stirred using a pestle to obtain a mixture. Thereafter, the above-obtained solution was added dropwise to this mixture, using a Pasteur pipette, and the thus obtained mixture was then stirred using a pestle. The obtained powders were transferred into an egg-plant shaped flask, and were then dried using an evaporator under reduced pressure for 3 hours, so as to produce the Everolimus-containing pharmaceutical composition of Comparative Example 4.

The pH of DL-α-tocopherol which was used as a stabilizer in Comparative Example 4 (at concentration of 2.5 mg/mL (in water/EtOH=1:3 mixture)) was measured by MM-60R (TOA DKK), and was found to be 8.2.

[Comparative Example 5]

70 mg of Everolimus was weighed into a test tube, and 100 μL of ascorbyl palmitate (manufactured by Wako Pure Chemical Industries, Ltd.) anhydrous ethanol solution (14 mg/mL) was then added thereto. The mixture was irradiated with ultrasonic wave for 10 minutes, and thereafter, dissolution of everolimus was confirmed. After that, 100 μL of anhydrous ethanol was added to the mixed solution to dilute it. Thereafter, 630 mg of anhydrous lactose (Super Tab (registered trademark) 21 AN, manufactured by DFE Pharma) and 70 mg of hypromellose (TC-5 E Type, manufactured by Shin-Etsu Chemical Co., Ltd.) were weighed into a mortar, and these substances were then stirred using a pestle to obtain a mixture. Thereafter, the above-obtained solution was added dropwise to this mixture, using a Pasteur pipette, and the thus obtained mixture was then stirred using a pestle. The obtained powders were transferred into an egg-plant shaped flask, and were then dried using an evaporator under reduced pressure for 3 hours, so as to produce the Everolimus-containing pharmaceutical composition of Comparative Example 5.

The pH of ascorbyl palmitate which was used as a stabilizer in Comparative Example 5 (at concentration of 5 mg/mL (in water/EtOH=1:1 mixture)) was measured by MM-60R (TOA DKK), and was found to be 3.4.

[Comparative Example 6]

70 mg of Everolimus was weighed into a test tube, and 200 μL of anhydrous ethanol was then added thereto. The mixture was irradiated with ultrasonic wave for 10 minutes, and thereafter, dissolution of everolimus was confirmed. 630 mg of anhydrous lactose (Super Tab (registered trademark) 21 AN, manufactured by DFE Pharma) and 70 mg of hypromellose (TC-5 E Type, manufactured by Shin-Etsu Chemical Co., Ltd.) were weighed into a mortar, and these substances were then stirred using a pestle to obtain a mixture. Thereafter, the above-obtained solution was added dropwise to this mixture, using a Pasteur pipette, and the thus obtained mixture was then stirred using a pestle. The obtained powders were transferred into an egg-plant shaped flask, and were then dried using an evaporator under reduced pressure for 3 hours, so as to produce the Everolimus-containing pharmaceutical composition of Comparative Example 6.

[Test Example 1]

The pharmaceutical compositions obtained by the methods applied in Examples 1 to 2 and Comparative Examples 1 to 6 (approximately 500 mg each) were each collected in a brown sample bottle, and were then preserved under light-shielded conditions at 60°C, in a desiccator, the humidity of which had been adjusted with a saturated cobalt chloride aqueous solution, while the bottle was uncapped.

The residual amounts of everolimus 7 days and 14 days after initiation of the preservation were measured by liquid chromatography (HPLC), and the residual percentages of everolimus at individual time points were then calculated. It is to be noted that the residual percentage was calculated according to the following formula. The results are shown in Table 2.

$$\text{Residual percentage (\%) of everolimus} = (\text{peak area of everolimus measured by HPLC at each time point/weighed value of powders}) / (\text{peak area of everolimus measured by HPLC before preservation (initial)/weighed value of powders}) \times 100$$

[Table 2]

|  | Additive | pH | Residual percentage (%) of everolimus | |
|---|---|---|---|---|
|  |  |  | Value on Day 3 | Value on Day 14 |
| Example 1 | sodium ascorbate | 8.4 | 97 | 82 |
| Example 2 | disodium hydrogen citrate | 6.3 | 94 | 66 |
| Comparative Example 1 | BHT | 8.6 | 98 | 60 |
| Comparative Example 2 | Ascorbic acid | 3.6 | 93 | 36 |
| Comparative Example 3 | Citric acid | 3.5 | 68 | 5 |
| Comparative Example 4 | DL-α-tocopherol | 8.2 | 85 | 35 |
| Comparative Example 5 | ascorbyl palmitate | 3.4 | 90 | 28 |
| Comparative Example 6 | None | -* | 88 | 28 |
| * not measured | | | | |

A decomposition reaction such as oxidation progresses in a pharmaceutical composition comprising everolimus at 60°C under humidified conditions, and as a result, the content of everolimus is reduced. In Test Example 1, in the case of the pharmaceutical composition comprising no antioxidant according to Comparative Example 6, a rapid reduction in the content of everolimus was observed. In the case of the pharmaceutical compositions comprising antioxidant such as ascorbic acid which is conventionally used as an antioxidant for pharmaceutical product according to Comparative Example 1 to 5, stabilization effect on everolimus was shown in some cases up to Day 3, but a rapid reduction in the content of everolimus was observed on Day 14. In contrast, in the case of the pharmaceutical composition according to Examples 1 and 2 of the present invention, wherein sodium ascorbate or disodium hydrogen citrate was added, the content of everolimus was high even 14 days after initiation of the preservation, and thus, it was found that the continuation of the stabilization effect was remarkably improved.

Ascorbic acid or citric acid are well-known as an antioxidant, but the formulation of Comparative Examples 2 and 3 using them did not show the preservation stabilization effect on everolimus. Therefore, it was shown to be necessary to use sodium salt of ascorbic acid or citric acid when ascorbic acid or citric acid is used as an antioxidant. Namely, it is considered to be necessary to use a salt having optimum pH in order to use ascorbic acid or citric acid as a stabilizer for everolimus.

A currently commercially available everolimus formulation (registered trademarks: Afinitor and Certican) contains dibutylhydroxytoluene (BHT). Therefore, the stabilization effect of Comparative Example 1 which is BHT containing composition corresponds to a standard of practical stability. Examples 1 and 2 of the present invention show the stability effect which is equal to or more superior to Comparative Example 1. It was revealed that Example 1 using sodium ascorbate showed surprisingly remarkable stabilization effect. It has been reported that BHT which is used as a stabilizer in Comparative Example 1 exhibits carcinogenicity or reproduction toxicity, and thus, this is a substance, the use amount of which is limited. On the other hand, ascorbate and citrate are broadly used as pharmaceutical product additives or food product additives. The toxicity problem is small and the limit of use amount is small, and they are highly safe additives. Accordingly, the pharmaceutical compositions of Examples 1 and 2 ensure high stabilization of everolimus as well as safety caused by additives.

[Example 3]

30 mg of Everolimus was weighed into a test tube, and 120 μL of ascorbyl stearate (manufactured by Tokyo Chemical Industry Co., Ltd.) anhydrous ethanol solution (5 mg/mL) and 60 μL of Meglumine (manufactured by LKT Laboratories) anhydrous ethanol solution (2.5 mg/mL) were then added thereto. The mixture was irradiated with ultrasonic wave for 10 minutes, and thereafter, dissolution of everolimus was confirmed. 270 mg of anhydrous lactose (Super Tab (registered trademark) 21 AN, manufactured by DFE Pharma) and 30 mg of hypromellose (TC-5 E Type, manufactured by Shin-Etsu Chemical Co., Ltd.) were weighed into a mortar, and these substances were then stirred using a pestle to obtain a mixture. Thereafter, the above-obtained solution was added dropwise to this mixture, using a Pasteur pipette, and the thus obtained mixture was then stirred using a pestle. The obtained powders were transferred into an egg-plant shaped flask, and were then dried using an evaporator under reduced pressure for 3 hours, so as to produce the Everolimus-containing pharmaceutical composition of Example 3.

The pH of solution obtained by dissolving 400 mg of ascorbyl stearate and 100 mg of Meglumine (which were used as stabilizer in Example 3) in 100 mL of mixture of water : EtOH = 1:1 was measured by MM-60R (TOA DKK), and was found to be 5.7.

[Example 4]

30 mg of Everolimus was weighed into a test tube, and 120 μL of ascorbyl stearate (manufactured by Tokyo Chemical Industry Co., Ltd.) anhydrous ethanol solution (5 mg/mL) and 120 μL of Meglumine (manufactured by LKT Laboratories) anhydrous ethanol solution (2.5 mg/mL) were then added thereto. The mixture was irradiated with ultrasonic wave for 10 minutes, and thereafter, dissolution of everolimus was confirmed. 270 mg of anhydrous lactose (Super Tab (registered trademark) 21 AN, manufactured by DFE Pharma) and 30 mg of hypromellose (TC-5 E Type, manufactured by Shin-Etsu Chemical Co., Ltd.) were weighed into a mortar, and these substances were then stirred using a pestle to obtain a mixture. Thereafter, the above-obtained solution was added dropwise to this mixture, using a Pasteur pipette, and the thus obtained mixture was then stirred using a pestle. The obtained powders were transferred into an egg-plant shaped flask, and were then dried using an evaporator under reduced pressure for 3 hours, so as to produce the Everolimus-containing pharmaceutical composition of Example 4.

The pH of solution obtained by dissolving 333.3 mg of ascorbyl stearate and 166.7 mg of Meglumine (which were used as stabilizer in Example 4) in 100 mL of mixture of water : EtOH = 1:1 was measured by MM-60R (TOA DKK), and was found to be 8.5.

[Example 5]

30 mg of Everolimus was weighed into a test tube, and 120 μL of ascorbyl stearate (manufactured by Tokyo Chemical Industry Co., Ltd.) anhydrous ethanol solution (5 mg/mL) and 240 μL of Meglumine (manufactured by LKT Laboratories) anhydrous ethanol solution (2.5 mg/mL) were then added thereto. The mixture was irradiated with ultrasonic wave for 10 minutes, and thereafter, dissolution of everolimus was confirmed. 270 mg of anhydrous lactose (Super Tab (registered trademark) 21 AN, manufactured by DFE Pharma) and 30 mg of hypromellose (TC-5 E Type, manufactured by Shin-Etsu Chemical Co., Ltd.) were weighed into a mortar, and these substances were then stirred using a pestle to obtain a mixture. Thereafter, the above-obtained solution was added dropwise to this mixture, using a Pasteur pipette, and the thus obtained mixture was then stirred using a pestle. The obtained powders were transferred into an egg-plant shaped flask, and were then dried using an evaporator under reduced pressure for 3 hours, so as to produce the Everolimus-containing pharmaceutical composition of Example 5.

The pH of solution obtained by dissolving 250 mg of ascorbyl stearate and 250 mg of Meglumine (which were used as stabilizer in Example 5) in 100 mL of mixture of water : EtOH = 1:1 was measured by MM-60R (TOA DKK), and was found to be 9.5.

[Comparative Example 7]

30 mg of Everolimus was weighed into a test tube, and 120 μL of dibutylhydroxytoluene (BHT, manufactured by MERCK) anhydrous ethanol solution (5 mg/mL) was then added thereto. The mixture was irradiated with ultrasonic wave for 10 minutes, and thereafter, dissolution of everolimus was confirmed. 270 mg of anhydrous lactose (Super Tab (registered trademark) 21 AN, manufactured by DFE Pharma) and 30 mg of hypromellose (TC-5 E Type, manufactured by Shin-Etsu Chemical Co., Ltd.) were weighed into a mortar, and these substances were then stirred using a pestle to obtain a mixture. Thereafter, the above-obtained solution was added dropwise to this mixture, using a Pasteur pipette, and the thus obtained mixture was then stirred using a pestle. The obtained powders were transferred into an egg-plant shaped flask, and were then dried using an evaporator under reduced pressure for 3 hours, so as to produce the Everolimus-containing pharmaceutical composition of Comparative Example 7.

The pH of BHT which was used as a stabilizer in Comparative Example 7 (at concentration of 2.5 mg/mL (in water/EtOH=1:3 mixture)) was measured by MM-60R (TOA DKK), and was found to be 8.6.

[Test Example 2]

The pharmaceutical compositions obtained by the methods applied in Examples 3 to 5 and Comparative Example 7 (approximately 300 mg each) were each collected in a brown sample bottle, and were then preserved under light-shielded conditions at 60°C, in a desiccator, the humidity of which had been adjusted with a saturated cobalt chloride aqueous solution, while the bottle was uncapped.

The residual amounts of everolimus 10 days and 14 days after initiation of the preservation were measured by liquid chromatography (HPLC), and the residual percentages of everolimus at individual time points were then calculated. It is to be noted that the residual percentage was calculated according to the following formula. The results are shown in Table 3.

Residual percentage (%) of everolimus = (peak area of everolimus measured by HPLC at each time point/weighed value of powders) / (peak area of everolimus measured by HPLC before preservation (initial)/weighed value of powders) x 100

[Table 3]

| | Additive | pH | Residual percentage (%) of Everolimus | |
| --- | --- | --- | --- | --- |
| | | | Value on Day 10 | Value on Day 14 |
| Example 3 | ascorbyl stearate + Meglumine | 5.7 | 89 | 84 |
| Example 4 | ascorbyl stearate + Meglumine | 8.5 | 92 | 82 |
| Example 5 | ascorbyl stearate + Meglumine | 9.5 | 91 | 86 |

(continued)

| | Additive | pH | Residual percentage (%) of Everolimus | |
|---|---|---|---|---|
| | | | Value on Day 10 | Value on Day 14 |
| Comparative Example 7 | BHT | 8.6 | 89 | 79 |

As a result, in Examples 3 to 5, the decomposition of everolimus was suppressed and everolimus was stabilized. The stabilization was continued and was stronger on Day 14 as compared with Comparative Example 7. Ascorbyl stearate is an acidic compound. However, a pharmaceutical composition which has higher stabilization effect as compared with the case of using BHT could be provided by setting the pH to be 5 to 10 by allowing ascorbyl stearate to co-exist with Meglumine which is amino sugar.

Aascorbyl stearate and Meglumine are broadly used as pharmaceutical product additives or food product additives. The toxicity problem is small and the limit of use amount is small, and they are highly safe additives. Accordingly, the pharmaceutical compositions of Examples 3 to 5 ensure high stabilization of everolimus as well as safety caused by additives.

## Claims

1. A pharmaceutical composition comprising (A) rapamycin or a derivative thereof, and (B) a salt of an ascorbic acid or a derivative thereof and/or a salt of chelating agent.

2. The pharmaceutical composition according to claim 1, which is produced by preparing a solution containing (A) rapamycin or a derivative thereof, and (B) a salt of an ascorbic acid or a derivative thereof and/or a salt of chelating agent, and then removing the solvent from the solution.

3. The pharmaceutical composition according to claim 1 o 2, wherein (B) the salt of an ascorbic acid or the derivative thereof and/or a salt of chelating agent has a pH of 4 to 11 in 5mg/mL 50%(v/v) ethanol aqueous solution.

4. The pharmaceutical composition according to any one of claims 1 to 3, which comprises 0.0001 to 20.0 parts by mass of (B) a salt of an ascorbic acid or a derivative thereof and/or a salt of chelating agent based on 1 part by mass of (A) the rapamycin or a derivative thereof.

5. The pharmaceutical composition according to any one of claims 1 to 4, which comprises a cellulose derivative and/or sugars.

6. A pharmaceutical formulation comprising the pharmaceutical composition according to any one of claims 1 to 5.

7. A method for producing a pharmaceutical composition comprising rapamycin or a derivative thereof, which comprises preparing a solution containing (A) rapamycin or a derivative thereof, and (B) a salt of an ascorbic acid or a derivative thereof and/or a salt of chelating agent, and then removing the solvent from the solution.

8. A method for producing a pharmaceutical formulation comprising rapamycin or a derivative thereof, which comprises preparing a solution containing (A) rapamycin or a derivative thereof, and (B) a salt of an ascorbic acid or a derivative thereof and/or a salt of chelating agent, then mixing the solution with a cellulose derivative and/or sugars, and then removing the solvent from the mixture.

9. A method for producing a pharmaceutical formulation comprising rapamycin or a derivative thereof, which comprises preparing a solution containing (A) rapamycin or a derivative thereof, and (B) a salt of an ascorbic acid or a derivative thereof and/or a salt of chelating agent, and then removing the solvent from the prepared solution to obtain a pharmaceutical composition comprising the rapamycin or a derivative thereof, and adding a cellulose derivative and/or sugars to said pharmaceutical composition.

**Amended claims under Art. 19.1 PCT**

1.  (Amended) A pharmaceutical composition comprising (A) rapamycin or a derivative thereof, and (B) a salt of an ascorbic acid or a derivative thereof and/or a salt of chelating agent, which is produced by preparing a solution containing (A) rapamycin or a derivative thereof, and (B) a salt of an ascorbic acid or a derivative thereof and/or a salt of chelating agent, and then removing the solvent from the solution.

2.  (Amended) The pharmaceutical composition according to claim 1, which comprises, as crystallized product of a solid mixture, (A) rapamycin or a derivative thereof, and (B) a salt of an ascorbic acid or a derivative thereof and/or a salt of chelating agent.

3.  The pharmaceutical composition according to claim 1 o 2, wherein (B) the salt of an ascorbic acid or the derivative thereof and/or a salt of chelating agent has a pH of 4 to 11 in 5mg/mL 50%(v/v) ethanol aqueous solution.

4.  The pharmaceutical composition according to any one of claims 1 to 3, which comprises 0.0001 to 20.0 parts by mass of (B) a salt of an ascorbic acid or a derivative thereof and/or a salt of chelating agent based on 1 part by mass of (A) the rapamycin or a derivative thereof.

5.  The pharmaceutical composition according to any one of claims 1 to 4, which comprises a cellulose derivative and/or sugars.

6.  A pharmaceutical formulation comprising the pharmaceutical composition according to any one of claims 1 to 5.

7.  A method for producing a pharmaceutical composition comprising rapamycin or a derivative thereof, which comprises preparing a solution containing (A) rapamycin or a derivative thereof, and (B) a salt of an ascorbic acid or a derivative thereof and/or a salt of chelating agent, and then removing the solvent from the solution.

8.  A method for producing a pharmaceutical formulation comprising rapamycin or a derivative thereof, which comprises preparing a solution containing (A) rapamycin or a derivative thereof, and (B) a salt of an ascorbic acid or a derivative thereof and/or a salt of chelating agent, then mixing the solution with a cellulose derivative and/or sugars, and then removing the solvent from the mixture.

9.  A method for producing a pharmaceutical formulation comprising rapamycin or a derivative thereof, which comprises preparing a solution containing (A) rapamycin or a derivative thereof, and (B) a salt of an ascorbic acid or a derivative thereof and/or a salt of chelating agent, and then removing the solvent from the prepared solution to obtain a pharmaceutical composition comprising the rapamycin or a derivative thereof, and adding a cellulose derivative and/or sugars to said pharmaceutical composition.

10. (Added) The method for producing a pharmaceutical composition according to claim 7, wherein (B) the salt of an ascorbic acid or the derivative thereof and/or a salt of chelating agent has a pH of 4 to 11 in 5mg/mL 50%(v/v) ethanol aqueous solution.

11. (Added) The method for producing a pharmaceutical formulation according to claim 8 or 9, wherein (B) the salt of an ascorbic acid or the derivative thereof and/or a salt of chelating agent has a pH of 4 to 11 in 5mg/mL 50%(v/v) ethanol aqueous solution.

12. (Added) The method for producing a pharmaceutical composition according to claim 7, wherein the pharmaceutical composition comprises 0.0001 to 20.0 parts by mass of (B) a salt of an ascorbic acid or a derivative thereof and/or a salt of chelating agent based on 1 part by mass of (A) the rapamycin or a derivative thereof.

13. (Added) The method for producing a pharmaceutical formulation according to claim 8 or 9, wherein the pharmaceutical composition comprises 0.0001 to 20.0 parts by mass of (B) a salt of an ascorbic acid or a derivative thereof and/or a salt of chelating agent based on 1 part by mass of (A) the rapamycin or a derivative thereof.

## INTERNATIONAL SEARCH REPORT

| International application No. |
| --- |
| PCT/JP2016/075617 |

**A. CLASSIFICATION OF SUBJECT MATTER**
See extra sheet.

According to International Patent Classification (IPC) or to both national classification and IPC

**B. FIELDS SEARCHED**

Minimum documentation searched (classification system followed by classification symbols)
A61K31/436, A61K47/22, A61K47/26, A61K47/38, A61P1/04, A61P1/16, A61P3/10,
A61P11/00, A61P13/12, A61P17/00, A61P17/06, A61P19/02, A61P21/04,
A61P25/00, A61P27/02, A61P29/00, A61P37/06

Documentation searched other than minimum documentation to the extent that such documents are included in the fields searched
Jitsuyo Shinan Koho        1922-1996   Jitsuyo Shinan Toroku Koho   1996-2016
Kokai Jitsuyo Shinan Koho  1971-2016   Toroku Jitsuyo Shinan Koho   1994-2016

Electronic data base consulted during the international search (name of data base and, where practicable, search terms used)
JSTPlus/JMEDPlus/JST7580(JDreamIII),
CAplus/REGISTRY/MEDLINE/EMBASE/BIOSIS(STN)

**C. DOCUMENTS CONSIDERED TO BE RELEVANT**

| Category* | Citation of document, with indication, where appropriate, of the relevant passages | Relevant to claim No. |
| --- | --- | --- |
| X<br>A | WO 2014/082286 A1  (HANGZHOU ZYLOX PHARMA CO.,<br>LTD.),<br>05 June 2014 (05.06.2014),<br>examples 5, 8, 9, 10<br>& JP 2016-500112 A      & US 2015/0051242 A1<br>& WO 2014/082286 A1      & EP 2809675 A1<br>& AU 2012395673 A1       & CA 2863243 A1<br>& KR 10-2015-0003156 A   & CN 104854112 A | 1,3,4,6<br>2,5,7-9 |

☒ Further documents are listed in the continuation of Box C.          ☐ See patent family annex.

| | |
| --- | --- |
| * Special categories of cited documents:<br>"A" document defining the general state of the art which is not considered    to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>    14 September 2016 (14.09.16) | Date of mailing of the international search report<br>    27 September 2016 (27.09.16) |
| --- | --- |
| Name and mailing address of the ISA/<br>    Japan Patent Office<br>    3-4-3,Kasumigaseki,Chiyoda-ku,<br>    Tokyo 100-8915,Japan | Authorized officer<br><br><br>Telephone No. |

Form PCT/ISA/210 (second sheet) (January 2015)

**INTERNATIONAL SEARCH REPORT**

International application No.

PCT/JP2016/075617

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | JP 2011-512383 A (Novartis AG.),<br>21 April 2011 (21.04.2011),<br>claims<br>& US 2011/0009340 A1<br>claims<br>& WO 2009/103727 A2 & EP 2254599 A2<br>& AU 2009216757 A1 & CA 2715822 A1<br>& MX 2010009104 A & CN 101945668 A<br>& KR 10-2010-0126386 A | 1,3,4,6<br>2,5,7-9 |
| X<br>Y | JP 2007-517879 A (Wyeth),<br>05 July 2007 (05.07.2007),<br>claims; examples<br>& US 2005/0152983 A1<br>examples; claims<br>& WO 2005/070393 A2 & EP 1701698 A2<br>& CA 2552595 A1 & KR 10-2006-0130162 A<br>& CN 1921861 A & AU 2004314213 A1<br>& MX PA06007829 A & RU 2006122517 A | 1,3-6<br>2,5,7-9 |
| X<br>Y | CN 102499929 A (FUJIAN INSTITUTE OF<br>MICROBIOLOGY),<br>20 June 2012 (20.06.2012),<br>paragraphs [0002], [0003]; examples 7, 10<br>(Family: none) | 1-8<br>2,5,7-9 |
| X<br>Y | JP 2009-520818 A (Wyeth),<br>28 May 2009 (28.05.2009),<br>claims<br>& US 2007/0142422 A1<br>claims<br>& WO 2007/075621 A1 & EP 1962819 A1<br>& AR 58561 A1 & KR 10-2008-0077989 A<br>& CA 2632239 A1 & CN 101340901 A<br>& RU 2008121713 A & AU 2006331874 A1 | 1,3,4,6<br>2,5,7-9 |
| X<br>A | JP 2013-523209 A (Lutonix, Inc.),<br>17 June 2013 (17.06.2013),<br>compound 6; examples 8, 9<br>& WO 2011/119159 A1<br>Formulation 6; examples 8, 9<br>& EP 2550030 A1 & AU 2010348988 A1<br>& CA 2793832 A1 & CN 102883753 A<br>& MX 2012011038 A & RU 2012145021 A | 1-4,6,7<br>5,8,9 |
| X<br>A | JP 2005-296665 A (Cordis Corp.),<br>27 October 2005 (27.10.2005),<br>claims; tables 11, 12<br>& US 2005/0232964 A1<br>tables 11, 12; claims<br>& EP 1586338 A2 & DE 602005026078 D1<br>& CA 2504258 A1 & AT 496643 T<br>& CA 2504258 A1 | 1,3,4,6<br>2,5,7-9 |

Form PCT/ISA/210 (continuation of second sheet) (January 2015)

**INTERNATIONAL SEARCH REPORT**

International application No.

PCT/JP2016/075617

C (Continuation).    DOCUMENTS CONSIDERED TO BE RELEVANT

| Category* | Citation of document, with indication, where appropriate, of the relevant passages | Relevant to claim No. |
|---|---|---|
| Y | JP 11-509223 A  (Novartis AG.),<br>17 August 1999 (17.08.1999),<br>example 1<br>& US 6004973 A<br>example 1<br>& WO 1997/003654 A2      & EP 839028 A2<br>& DE 69631422 T2        & FR 2736550 A1<br>& RU 2159107 C2         & CN 1195289 A<br>& KR 10-0352943 B1 | 2,5,7,8 |
| Y | JP 2014-528431 A  (Novartis AG.),<br>27 October 2014 (27.10.2014),<br>example 7<br>& US 2014/0242162 A1<br>example 7<br>& WO 2013/050419 A1      & EP 2763663 A1<br>& TW 201318625 A         & AU 2012320563 A1<br>& CA 2850995 A1          & CN 103874484 A<br>& KR 10-2014-0058670 A   & MX 2014004166 A | 2,5,7,8 |
| Y | JP 2013-528225 A  (Abbot Laboratories),<br>08 July 2013 (08.07.2013),<br>example 4<br>& WO 2011/156578 A1<br>example 4<br>& US 2012/0258909 A1     & EP 2579854 A1<br>& MX 2012014384 A        & KR 10-2013-0053440 A<br>& CN 103209686 A | 2,5,7,9 |
| Y | WO 2014/076568 A2  (DR. REDDY'S LABORATORIES LTD.),<br>22 May 2014 (22.05.2014),<br>example 4<br>& JP 2016-503420 A       & EP 2919765 A2<br>& CA 2891502 A1          & AU 2013346501 A1<br>& CN 104918608 A         & MX 2015006223 A<br>& KR 10-2015-0084873 A | 2,5,7,9 |

Form PCT/ISA/210 (continuation of second sheet) (January 2015)

**INTERNATIONAL SEARCH REPORT**

International application No.

PCT/JP2016/075617

Continuation of A. CLASSIFICATION OF SUBJECT MATTER
  (International Patent Classification (IPC))

*A61K31/436*(2006.01)i, *A61K47/22*(2006.01)i, *A61K47/26*(2006.01)i,
*A61K47/38*(2006.01)i, *A61P1/04*(2006.01)i, *A61P1/16*(2006.01)i,
*A61P3/10*(2006.01)i, *A61P11/00*(2006.01)i, *A61P13/12*(2006.01)i,
*A61P17/00*(2006.01)i, *A61P17/06*(2006.01)i, *A61P19/02*(2006.01)i,
*A61P21/04*(2006.01)i, *A61P25/00*(2006.01)i, *A61P27/02*(2006.01)i,
*A61P29/00*(2006.01)i, *A61P37/06*(2006.01)i

(According to International Patent Classification (IPC) or to both national
classification and IPC)

Form PCT/ISA/210 (extra sheet) (January 2015)

**EP 3 345 601 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 11509223 A
- JP 2005507897 A
- JP 2002531527 A
- WO 2013022201 A
- WO 94022136 A
- US 5258389 A
- WO 9409010 A
- WO 9205179 A
- US 5118677 A
- US 5100883 A
- US 5151413 A